# EUROPEAN PATENT SPECIFICATION

(11) **EP 1 385 577 B1**
(45) Date of publication and mention of the grant of the patent: **19.04.2006**
(21) Application number: 02719751.6
(22) Date of filing: 02.02.2002
(51) Int. Cl.: A61P 13/08, A61K 31/44, A61K 31/455

(54) **USE OF NK-1 RECEPTOR ANTAGONISTS AGAINST BENIGN PROSTATIC HYPERPLASIA**
VERWENDUNG VON NK-1-REZEPTORANTAGONISTEN GEGEN BENIGNE PROSTATAHYPERPLASIE
UTILISATION D'ANTAGONISTES DE RECEPTEUR DE NK-1 CONTRE L'HYPERPLASIE BENIGNE DE LA PROSTATE

(30) Priority: 23.04.2001 EP 01109853
(43) Date of publication of application: 04.02.2004
(73) Proprietor: F. Hoffman-la Roche AG, 4070 Basle (CH)
(72) Inventor: BUSER, Susanne, CH-4402 Frenkendorf (CH); FORD, Anthony, P., D., W., Mountain View, CA 94041 (US); HOFFMANN, Torsten, 79576 Weil am Rhein (DE); LENZ, Barbara, 79189 Bad Krozingen (DE); SLEIGHT, Andrew John, 68400 Riedisheim (FR); VANKAN, Pierre, CH-4054 Basel (CH)
(74) Representative: Wächter, Dieter Ernst
(86) International application number: PCT/EP2002/001085
(87) International publication number: WO 2002/085458

(56) References cited:
- EP-A- 1 035 115
- WO-A-00/67742
- WO-A-99/07677
- WO-A-99/07681
- GB-A- 2 347 422
- US-A- 5 859 029
- BULJUBASICH S ET AL: "An immunohistochemical and pharmacological study of tachykinins in the rat and guinea-pig prostate glands." EUROPEAN JOURNAL OF PHARMACOLOGY. NETHERLANDS 10 SEP 1999, vol. 380, no. 2-3, 10 September 1999 (1999-09-10), pages 137-144, XP001086473 ISSN: 0014-2999

## Description

The present invention concerns NK-1 receptor antagonists and their use for the treatment and/or prevention of benign prostatic hyperplasia (BPH).

Benign prostatic hyperplasia (BPH) is quite common in older men. Its symptoms may interfere with daily activities and impact the perception of wellbeing and thus the quality of life. BPH can be progressive and lead to urinary retention, infections, bladder calculi and renal failure. While moderate symptoms may remain untreated, bothersome symptoms and complications may need medical therapy or surgery.

Catheterization may be needed in case of an acute urinary retention, one of the complications caused by BPH. There are two different forms of acute urinary retention, viz. spontaneous or precipitated acute urinary retention, whereby the first one is often considered by patients to be the most serious outcome of BPH. Spontaneous acute urinary retention can be treated with 5-alpha-reductase inhibitors, such as finasteride as described by Andersen et al., Urology, 49(6), 839-845, (1997). Precipitated acute urinary retention is an episode of acute urinary retention which often occurs within the first three days after anesthesia or surgery, after a stroke or a congestive heart failure; a medical condition such as prostatitis or urinary tract infection; or ingestion of medication or drugs known to precipitate retention, e.g., pseudoephedrine hydrochloride, cold medicine, pain medication such as narcotics or sedatives, or benadryl.

Benign prostatic hyperplasia (BPH) is unusual in that it occurs spontaneously as a clinical disease in males of only two species, humans and dogs (Emberton M. and Mundy A.R. (1999), "The Prostate and Benign Prostatic Hyperplasia", in The Scientific Basis of Urology, editors Mundy, Fitzpatrick, Neal & George; Isis Medical Media, Oxford UK. 257pp.). Anatomical similarities between canine and human prostate were first extensively reviewed by Price D. in "Comparative aspects of development and structure in the prostate". Natl. Cancer Inst. Monogr., 12, 1-7, (1963), through developmental studies: the canine prostate surrounds the neck of the bladder and proximal urethra, grossly resembling the human prostate, is of mixed stromal and glandular morphology and is ensheathed in a capsule of smooth muscle, fibrovascular tissue, nerves and ganglia. In BPH of dogs and in men, the epithelial and stromal prostatic elements both increase in amount in a seemingly uncoordinated fashion (see Strandberg J.D. in "Comparative Pathology of Benign Prostatic Hyperplasia", in, Prostatic Diseases, editor Lepor H., W.B. Saunders Company, Philadelphia (2000)). Accordingly, dogs have been used extensively in experimental studies of the etiology, pathogenesis and treatment of BPH (Walsh P.C. and Wilson J.D., "The induction of prostatic hypertrophy in the dog with androstanediol", J. Clin. Invest., 57, 1093-7, (1976); Suzuki K., Okazaki H., Ono Y., Kurokawa K., Suzuki T., Onuma E., Takanashi H., Mamiya Y. and Yamanaka H., "Effect of dual inhibition of 5-a-reductase and aromatase on spontaneously developed canine prostatic hypertrophy", Prostate (NY), 37(2), 70-76, (1998); for a review see also Strandberg J.D. (2000; cited above).

Neurokinin-1 (NK-1) or substance P is a naturally occurring undecapeptide belonging to the tachykinin family of peptides, the latter being so-named because of their prompt contractile action on extravascular smooth muscle tissue. The receptor for neurokinin-1 or substance P is a member of the superfamily of G protein-coupled receptors and is named NK-1 receptor. This receptor is widely distributed throughout the mammalian nervous system (especially brain and spinal ganglia) and is also present in the circulatory system and in peripheral tissues (especially the duodenum, the jejunum and the genito-urinary tract). The receptor is believed to be involved in the regulation of a number of diverse biological processes as outlined below.

The central and peripheral actions of the mammalian tachykinin substance P have been associated with numerous inflammatory conditions including migraine, rheumatoid arthritis, asthma, and inflammatory bowel disease as well as mediation of the emetic reflex and the modulation of central nervous system (CNS) disorders such as Parkinson's disease (Neurosci. Res., 7, 187-214, (1996)), anxiety (Can. J. Phys., 75, 612-621, (1997)) and depression (Science, 281, 1640-1645, (1998)).

Evidence for the usefulness of tachykinin receptor antagonists in pain, headache, especially migraine, Alzheimer's disease, multiple sclerosis, attenuation of morphine withdrawal, cardiovascular changes, edema, such as edema caused by thermal injury, chronic inflammatory diseases such as rheumatoid arthritis, asthma/bronchial hyperreactivity and other respiratory diseases including allergic rhinitis, inflammatory diseases of the gut including ulcerative colitis and Crohn's disease, ocular injury and ocular inflammatory diseases reviewed in "Tachykinin Receptor and Tachykinin Receptor Antagonists", J. Auton. Pharmacol., 13, 23-93, (1993).

Furthermore, neurokinin-1 receptor antagonists are being developed for the treatment of a number of physiological disorders associated with an excess or imbalance of tachykinin, in particular substance P. Examples of conditions in which substance P has been implicated include disorders of the central nervous system such as anxiety, depression and psychosis (International Patent Application, Publication Nos. WO 95/16679, WO 95/18124 and WO 95/23798).

The neurokinin-1 receptor antagonists are further believed to be useful for the treatment of motion sickness and for treatment induced vomiting.

The reduction of cisplatin-induced emesis by a selective neurokinin-1-receptor antagonist is described in The New England Journal of Medicine, Vol. 340, No. 3, 190-195, (1999).

Furthermore, US Patent No. 5,972,938 describes a method for treating a psychoimmunologic or a psychosomatic disorder by administration of a tachykinin receptor, such as the NK-1 receptor antagonist.

The usefulness of neurokinin 1 receptor antagonists for the treatment of certain forms of urinary incontinence is furthermore described in Neuropeptides, 32(1), 1-49, (1998) and Eur. J. Pharmacol., 383(3), 297-303, (1999).

NK-1 receptor antagonists have been reported to have also a beneficial effect in the therapy of traumatic brain injury (oral disclosure by Prof. Nimmo at the International Tachykinin Conference 2000 in La Grande Motte, France, October 17-20, 2000 with the title "Neurokinin 1 (NK-1) Receptor Antagonists Improve the Neurological Outcome Following Traumatic Brain Injury", Authors: Nimmo A.J., Bennett C.J., Hu X., Cernak I., Vink R.).

The use of NK-1 receptor antagonists for the treatment or prevention of chronic nonbacterial prostatitis and prostatodynia has been described in International Patent Publication No. WO 99/59583.

International Patent Publication No. WO O1/01922 describes the use of substance P antagonists in the treatment of the adenocarcinomas, particularly genito-urinary tract neoplasms such as prostatic carcinoma.

WO99/07681 refers to the treatment and prevention ofirritative symptoms (emphasis added) of benign prostatic hypertrophy and not to the treatment of BPH per se. Moreover, the said reference to irritative symptoms of benign prostatic hyperplasia is part of a long list of diseases including many dozens of diseases, wherein the preferred treatment is pain.

WO99/07677 describes similar compounds as in WO99/07681 and again refers to the treatment and prevention of irritative symptoms (emphasis added) of benign prostatic hypertrophy and not to the treatment of BPH per se.

WO00/67742 refers to the treatment or prevention of benign prostatic hypertrophy and not to the treatment of BPH per se.

GB 2347422 discloses novel NK-1 receptor antagonists, but do not disclose their use in the treatment and prevention of BPH.

The "European Journal of Pharmacology, NL, 1999, Vol 380, No. 2 - 3, pp 137 - 144" discloses immunohistochemical and pharmacological studies of tachykinin in the rat and guineapig prostate glands. It does not provide data on the growth and hyperplasia of the glandular or stromal tissues.

The terms "NK-1 receptor antagonist" and "Substance P receptor antagonist" are used herein refer to any synthetic chemical compound that inhibits binding of substance P to the NK-1 receptor. A large number of such receptor antagonists are known and have been described e.g. in European Patent Publication No. EP-A-1,035,115 of Boes M., Branca Q., Galley G., Godel T., Hoffmann T., Hunkeler W., Schnider P., Stadler H., entitled "Preparation of N-benzyl-4-tolylnicotinamides and related compounds as neurokinin-1 receptor antagonists.".

It has now been found that the selective NK-1 receptor antagonist of the general formula 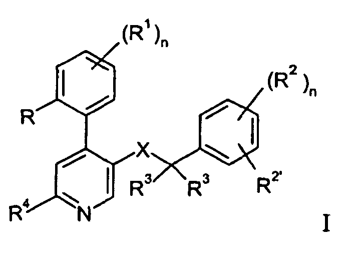 wherein
- R: is hydrogen, C₁₋₇-alkyl, C₁₋₇-alkoxy, halogen or trifluoromethyl;
- R¹: is hydrogen or halogen; or
- R and R¹: may be together -CH=CH-CH=CH-;
- R² and R^{2'}: are independently from each other hydrogen, halogen, trifluoromethyl, C₁₋₇-alkoxy or cyano; or
- R² and R^{2'}: may be together -CH=CH-CH=CH-, optionally substituted by one or two substituents selected from C₁₋₇-alkyl or C₁₋₇-alkoxy;
- R³: is hydrogen, C₁₋₇-alkyl or form a C₃₋₆-cycloalkyl group;
- R⁴: is hydrogen, -N(R⁵)₂, -N(R⁵)(CH₂)ₙOH, -N(R⁵)S(O)₂- C₁₋₇-alkyl, -N(R⁵)S(O)₂-phenyl, -N=CH-N(R⁵)₂, -N(R⁵)C(O)R⁵ or a cyclic tertiary amine of the group 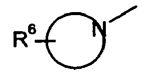
or the group 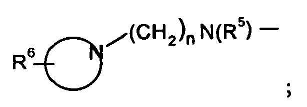
- R⁵: is, independently from each other, hydrogen, C₃₋₆-cycloalkyl, benzyl or C₁₋₇-alkyl;
- R⁶: is hydrogen, hydroxy, C₁₋₇-alkyl, -(CH₂)ₙCOO-C₁₋₇-alkyl, -N(R⁵)CO- C₁₋₇-alkyl, hydroxy-C₁₋₇-alkyl, cyano, -(CH₂)ₙO(CH₂)ₙOH, -CHO or a 5-or 6 membered heterocyclic group, optionally bonded via an -(CH₂)₁₋₇-group;
- X: is -C(O)N(R⁵)- or -N(R⁵)C(O)-,;
- n: is 0, 1, 2, 3 or 4;
and the pharmaceutically acceptable acid addition salts thereof are particularly suitable for the treatment and/or prevention of benign prostatic hyperplasia.

The present invention also relates to the use of an NK-1 receptor antagonist of the general formula (1) for the manufacture of a medicament for the treatment and/or prevention of benign prostatic hyperplasia.

The following definitions of the general terms used in the present description apply irrespective of whether the terms in question appear alone or in combination.

The term "halogen" denotes chlorine, iodine, fluorine and bromine.

The term "cyclic tertiary amine" denotes, for example, pyrrolidin-1-yl, imidazol-1-yl, piperidin-1-yl, piperazin-1-yl, morpholin-4-yl, thiomorpholin-4-yl, 1-oxo-thiomorpholin-4-yl, 1,1-dioxo-thiomorpholin-4-yl, 2,3-dihydro-[1,4]oxazin-4-yl, or [1,2,4]triazol-1-yl.

The term "five or six membered heteroaryl group, containing one to four heteroatoms, selected from N, O or S" denotes, for example, the following groups: pyrrol-1-yl, imidazol-1 or 2-yl, pyrazol-1-yl, pyridin-2, 3 or 4-yl, pyrazinyl, pyrimidinyl, pyridazinyl, isothiazolyl, isoxazolyl, thienyl, 1,2,3-triazolyl, 1,2,4-oxadiazolyl, tetrahydropyridinyl, isoxazolyl or furyl.

The term "five or six membered saturated cyclic tertiary amine" denotes, for example, pyrrolidinyl, imidazolidinyl, pyrazolidinyl, piperidinyl, piperazinyl, morpholinyl, thiomorpholinyl, thiomorpholin-1,1-dioxo or thiomorpholin-1-oxo.

The term "5 or 6 membered heterocyclic group" denotes, for example pyridinyl, pyrimidinyl, oxadiazolyl, triazolyl, tetrazolyl, thiazolyl, thienyl, furyl, pyranyl, pyrrolyl, imidazolyl, pyrazolyl, isothiazolyl, piperazinyl or piperidyl.

The term "aryl" denotes a monocyclic aromatic hydrocarbon radical or a bicyclic or tricyclic ring system in which at least one ring is aromatic, preferred are phenyl, benzyl or naphthyl rings.

The term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methanesulfonic acid, p-toluenesulfonic acid and the like.

Preferred compounds for the claimed use are the exemplary compounds in which X in general formula (I) is -C(O)N(R⁵)- and wherein R⁵ is methyl, ethyl or cyclopropyl, for example the following compounds:
N-(3,5-bis-trifluoromethyl-benzyl)-N-methyl-4-o-tolyl-nicotinamide,
N-(3,5-bis-trifluoromethyl-benzyl)-N-methyl-4-(2-chloro-phenyl)-nicotinamide,
N-(3,5-bis-trifluoromethyl-benzyl)-N-methyl-4-(2-trifluoromethyl-phenyl)-nicotinamide,
N-(3,5-bis-trifluoromethyl-benzyl)-N-methyl-4-(2-fluoro-phenyl)-nicotinamide,
N-(3,5-bis-trifluoromethyl-benzyl)-N-methyl-4-(2-methoxy-phenyl)-nicotinamide,
N-(3,5-bis-trifluoromethyl-benzyl)-N-methyl-4-phenyl-nicotinamide,
N-(3,5-bis-trifluoromethyl-benzyl)-N-ethyl-4-o-tolyl-nicotinamide,
N-(3,5-bis-trifluoromethyl-benzyl)-N-cyclopropyl-4-o-tolyl-nicotinamide,
N-[1-(3,5-bis-trifluoromethyl-phenyl)-ethyl]-N-methyl-4-o-tolyl-nicotinamide,
N-(3,5-di-fluorobenzyl)-N-methyl-4-o-tolyl-nicotinamide,
N-(3,5-di-chlorobenzyl)-N-methyl-4-o-tolyl-nicotinamide,
N-(3,5-bis-trifluoromethyl-benzyl)-N-methyl-6-(4-methyl-piperazin-1-yl)-4-0-tolyl-nicotinamide,
N-(3,5-bis-trifluoromethyl-benzyl)-N-methyl-6-(4-methyl-piperazin-1-yl)-4-naphthalen-1-yl-nicotinamide,
(4-{5- [(3,5-bis-trifluoromethyl-benzyl)-methyl-carbamoyl]-4-o-tolyl-pyridin-2-yl}-piperazin-1-yl)-acetic acid ethyl ester,
5'- [(3,5-bis-trifluoromethyl-benzyl)-methyl-carbamoyl] -4'-o-tolyl-3,4,5,6-tetrahydro-2H-[1,2']bipyridinyl-4-carboxylic acid ethyl ester,
N-(3,5-bis-trifluoromethyl-benzyl)-N-methyl-6-(4-propyl-piperazin-1-yl)-4-0-tolyl-nicotinamide,
(RS)-6- [3-(acetyl-methyl-amino)-pyrroridin-1-yl]-N-(3,5-bis-trifluoromethylbenzyl)-N-methyl-4-o-tolyl-nicotinamide,
N-(3,5-bis-trifluoromethyl-benzyl)-N-methyl-6-[methyl-(2-morpholin-4-yl-ethyl)-amino]-4-o-tolyl-nicotinamide,
N-(3,5-bis-trifluoromethyl-benzyl)-N-methyl-6-morpholin-4-yl-4-o-tolyl-nicotinamide,
N-(3,5-bis-trifluoromethyl-benzyl)-N-methyl-6-thiomorpholin-4-yl-4-o-tolyl-nicotinamide,
N-(3,5-bis-trifluoromethyl-benzyl)-N-methyl-6-(1-oxo-1λ⁴-thiomorpholin-4-yl)-4-o-tolyl-nicotinamide,
N-(3,5-bis-trifluoromethyl-benzyl)-6-(1,1-dioxo-1λ⁶ -thiomorpholin-4-yl)-N-methyl-4-o-tolyl-nicotinamide,
N-(3,5-bis-trifluoromethyl-benzyl)-N-methyl-6-piperazin-1-yl-4-o-tolyl-nicotinamide,
N-(3,5-bis-trifluoromethyl-benzyl)-6-[4-(2-hydroxy-ethyl)-piperazin-1-yl]-N-methyl-4-o-tolyl-nicotinamide,
N-(3,5-bis-trifluoromethyl-benzyl)-6-(4-cyanomethyl-piperazin-1-yl)-N-methyl-4-o-tolyl-nicotinamide,
N-(3,5-bis-trifluoromethyl-benzyl)-6-{4-[2-(2-hydroxy-ethoxy)-ethyl]-piperazin-1-yl}-N-methyl-4-o-tolyl-nicotinamide,
N-(3,5-bis-trifluoromethyl-benzyl)-N-methyl-6-(4-[1,2,4]oxadiazol-3-yl-methyl-piperazin-1 -yl)-4-o-tolyl-nicotinamide,
N-(3,5-bis-trifluoromethyl-benzyl)-N-methyl-6-[4-(5-oxo-4,5-dihydro-1H-[1,2,4]triazol-3-yl-methyl)-piperazin-1-yl]-4-o-tolyl-nicotinamide,
N-(3,5-bis-trifluoromethyl-benzyl)-6-(4-formyl-piperazin-1-yl)-N-methyl-4-o - tolyl-nicotinamide, and
N-methyl-N-(2-methyl-naphthalen-1-yl-methyl)-6-morpholin-4-yl-4-o-tolyl-nicotinamide.

Further preferred compounds for the claimed use are the exemplary compounds in which X in general formula (I) is -N(R⁵)-CO- and wherein R⁵ is hydrogen or methyl, for example the following compounds:
2-(3,5-bis-trifluoromethyl-phenyl)-N-methyl-N-[6-(4-methyl-piperazin-1-yl)-4-o-tolyl-pyridin-3-yl]-isobutyramide,
2-(3,5-bis-trifluoromethyl-phenyl)-N-[4-(2-chloro-phenyl)-6-(4-methyl-piperazin-1-yl)-pyridin-3-yl]-N-methyl-isobutyramide,
2-(3,5-bis-trifluoromethyl-phenyl)-N-[4-(4-fluoro-2-methyl-phenyl)-6-(4-methyl-piperazin=1-yl)-pyridin-3-yl]-N-methyl-isobutyramide,
2-(3,5-bis-trifluoromethyl-phenyl)-N-[4-(2-chloro-phenyl)-pyridin-3-yl]-N-methyl-isobutyramide,
2-(3,5-bis-trifluoromethyl-phenyl-N-methyl-N-(4-o-tolyl-pyridin-3-yl)-isobutyramide,
2-(3,5-bis-trifluoromethyl-phenyl)-N-(4-o-tolyl-pyridin-3-yl)-isobutyramide,
2-(3,5-bis-trifluoromethyl-phenyl)-N-methyl-N-(4-o-tolyl-pyridin-3-yl)-acetamide,
2-(3,5-bis-trifluoromethyl-phenyl)-N-methyl-N-(4-o-tolyl-pyridin-3-yl)-propionamide,
2-(3,5-bis-trifluoromethyl-phenyl)-N-methyl-N-(6-morpholin-4-yl-4-o-tolyl-pyridin-3-yl)-isobutyramide,
2- 3,5-bis-trifluoromethyl-phenyl)-N- [4-(2-chloro-phenyl)-6-morpholin-4-yl-pyridin-3-yl]-N-methyl-isobutyramide,
2- 3,5-bis-trifluoromethyl-phenyl)-N-methyl-N-{6-[methyl-(2-morpholin-4-yl-ethyl)-amino]-4-o-tolyl-pyridin-3-yl)-isobutyramide,
2-(3,5-bis-trifluoromethyl-phenyl)-N-methyl-N-[6-(4-pyrimidin-2-yl-piperazin-1-yl)-4-o-tolyl-pyridin-3-yl]-isobutyramide,
2-(3,5-bis-trifluoromethyl-phenyl)-N-(6-morpholin-4-yl-4-o-tolyl-pyridin-3-yl)-isobutyramide,
2-(3,5-bis-trifluoromethyl-phenyl)-N- [4-(2-chloro-phenyl)-6-dimethylamino-pyridin-3-yl]-isobutyramide,
2-(3,5-bis-trifluoromethyl-phenyl)-N-methyl-N-(6-piperazin-1-yl-4-o-tolyl-pyridin-3-yl)-isobutyramide,
2-(3,5-bis-trifluoromethyl-phenyl)-N-(4-hydroxy-4'-o-tolyl-3,4,5,6-tetrahydro-2H-[1,2']bipyridinyl-5'-yl)-N-methyl-isobutyramide,
2-(3,5-bis-trifluoromethyl-phenyl)-N-{6-[(2-hydroxy-ethyl)-methyl-amino]-4-o-tolyl-pyridin-3-yl}-N-methyl-isobutyramide,
(R)-2-(3,5-bis-trifluoromethyl-phenyl)-N-[6-(3-hydroxy-pprolidin-1-yl)-4-o-tolyl-pyridin-3-yl] -N-methyl-isobutyramide,
2-(3,5-bis-trifluoromethyl-phenyl)-N-methyl-N-(6-morpholin-4-yl-4-o-tolyl-pyridin-3-yl)-acetamide, and
[2-(3,5-bis-trifluoromethyl-phenyl)-2-methyl-propyl]-[4-(4-fluoro-2-methylphenyl)-6-(4-methyl-piperazin-1-yl)-pyridin-3-yl]-methylamine.

The methods for the preparation of the above-mentioned compounds are described in detail in EP-A-1,035,115. Also provided are values for the affinity of selected compounds to the NK-1 receptor, given as pKi, whereby the pKi value for preferred compounds is in the range of 8.00 to 9.80. EP-A-1,035,115 provides furthermore proposals for suitable formulations of NK-1 receptor antagonists, which are also suitable for the use as claimed in the present patent specification.

Most preferred compound for the use in accordance with the present invention are 2-(3,5-bis-trifluoromethyl-phenyl)-N-methyl-N-(6-morpholin-4-yl-4-o-tolyl-pyridin-3-yl)-isobutyramide and 2-(3,5-bis-trifluoromethyl-phenyl)-N-methyl-N-[6-(4-methyl-piperazin-1-yl)-4-o-tolyl-pyridin-3-yl]-isobutyramide disclosed in EP-A-1,035,115, as well as 2-(3,5-bis-trifluoromethyl-phenyl)-N-[6-( 1,1-dioxo-1λ⁶-thiomorpholin-4-yl)-4-o-tolyl-pyridin-3-yl] -N-methyl-isobutyramide and 2-(3,5-bis-trifluoromethyl-phenyl)-N-(6-(1,1-dioxo-1λ⁶⁻thiomorpholin-4-yl)-4-(4-fluoro-2-methyl-phenyl)-pyridin-3-yl]-N-methyl-isobutyramide described in EP 1414525.

As mentioned above benign prostatic hyperplasia (BPH) or prostate hypertrophy is a disease of males, the incidence of which increases considerably after the fifth decade in the life of human beings. It is still not clear what causes BPH, but it appears that BPH is related to the hormone testosterone and its relationship to other hormones that change during the aging process. The fact that the prostate begins to grow larger is not necessarily a problem. In fact, some men have extremely enlarged prostates but suffer no ill effects. On the other hand, some men have prostates that are only slightly enlarged and they suffer from bothersome urinary symptoms. These symptoms include difficulties in urinating, the need to urinate quite frequently, or awaking during the night to urinate.

In serious cases BPH will either be treated through medical therapy using prescription medications or by surgical treatment to remove tissue that is obstructing the flow of urine. Therapy by prescription medication is preferred because it is non-invasive. A number of prescription medications for the treatment of BPH are known, such as e.g. the gonadotrophin agonist leuprorelin sold inter alia under the tradenames Lupron™ and Lupron Depot™ and the 5-alpha reductase inhibitor finasteride sold under the trademark of Proscar™. The present invention provides a novel class of prescription medication for the treatment of BPH, viz. NK-1 receptor antagonists.

NK-1 receptor antagonists for use in connection with the claimed invention may be administered either alone or in combination with other therapeutic agents and are preferably formulated to a pharmaceutical composition comprising pharmaceutically acceptable carriers or diluents. The pharmaceutical preparations to be used in accordance with this invention can in addition also contain preservatives, solubilizers, stabilizers, wetting agents, emulsifiers, sweeteners, colorants, flavorants, salts for varying the osmotic pressure, buffers, masking agents or antioxidants.

NK-1 receptor antagonists can be formulated in the form of a Self-Emulsifying Drug Delivery Systems (SEDDS), which consist of mixtures of oils and surfactants, ideally isotropic, which sometimes include co-solvents. Such mixtures emulsify under conditions of gentle agitation, similar to those which would be encountered in the gastro intestinal tract. When such a formulation is released into the lumen of the gut, it disperses to form a fine emulsion, so that the drug contained in the emulsion remains in solution in the gut, avoiding the dissolution step which frequently limits the rate of absorption of hydrophobic drugs from the crystalline state. SEDDS lead to improved bioavailability and/or a more consistent temporal profile of absorption from the gut. SEDDS have been described by Pouton C.W., in Advanced Drug Delivery Reviews, 25,(1997),47-58.

The NK-1 receptor antagonist or the pharmaceutical composition comprising it is preferably administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions. The NK-1 receptor antagonist or the pharmaceutically composition comprising it can also be administered via any other suitable way known to the person skilled in the art.

The dosage can vary within wide limits and can, of course, be fitted to the individual requirements in each particular case. The dosage range for a beneficial effect in mammals depends of course on the activity of the NK-1 receptor antagonist that is used, but is usually in the range of 5 to 1000 mg/kg/d and is preferably between 25 and 100 mg/kg/d.

An injection solution may have the following composition:

| Compound of formula (I) | 1 mg |
|---|---|
| 1 n HCl | 20 µl |
| acetic acid | 0.5 mg |
| NaCl | 8 mg |
| phenol | 10 mg |
| 1 n NaOH | q.s. ad pH 5 |
| H₂O | q.s. ad 1 ml |

The pharmaceutical preparations in accordance with this invention can in addition also contain pharmaceutically inert, inorganic or organic excipients suitable for the production of tablets, coated tablets, dragees and hard gelatine capsules. Lactose, corn starch or derivatives thereof, talc, stearic acid or its salts etc. can be used as such excipients e.g. for tablets, dragées and hard gelatine capsules.

Suitable excipients for soft gelatine capsules are e.g. vegetable oils, waxes, fats, semi-solid and liquid polyols etc.

Suitable excipients for the manufacture of solutions and syrups are e.g. water, polyols, saccharose, invert sugar, glucose etc.

Suitable excipients for injection solutions are e.g. water, alcohols, polyols, glycerol, vegetable oils etc.

Suitable excipients for suppositories are e.g. natural or hardened oils, waxes, fats, semiliquid or liquid polyols etc.

Moreover, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

As indicated in the following example below the inventors have shown that NK-1 receptor antagonists, in particular 2-(3,5-bis-trifluoromethyl-phenyl)-N-methyl-N-(6-morpholin-4-yl-4-o-tolyl-pyridin-3-yl)-isobutyramide, have the potential to reduce the size of prostate and can therefore be used in the treatment and/or prevention of benign prostatic hyperplasia.

### EXAMPLE

### Summary on a 39-week Toxicity Study in the Dog

In a nine-month study four groups of Beagle dogs (4 animals/gender/group; 5-6 months of age at study start) received oral doses (gavage) of 0 (placebo), 6, 20 and 60 mg/kg/d of 2-(3,5-bis-trifluoromethyl-phenyl)-N-methyl-N-(6-morpholin-4-yl-4-o-tolyl-pyridin-3-yl)-isobutyramide as a SEDDS formulation for 39 weeks. The following variables were investigated: clinical signs, body weights, food consumption, ophthalmoscopy before and at the end of the study, electrocardiography, heart rate, toxicokinetics at different time-points, clinical pathology (hematology, biochemistry, urinalysis) in 3-months intervals, necropsy and tissue preservation, organ weights and histopathology.

Dose-related reduced weights of the prostate gland were measured in males at 60 and 20 mg/kg/d (by 58% and 37% when compared to the control) with an insignificant trend also at 6 mg/kg/d. Microscopic changes were limited to all males at 60 and most at 20 mg/kg/d.

The finding was characterized by a smaller overall cross-sectional area, smaller acinar lumina and flatter epithelium with less eosinophilic cytoplasm, particularly in the center of the prostate. However, mitoses were evident in the peripheral acini. The prostate of low dose males was similar to controls.

It is known that the canine prostate exhibits regional differences in the response of the prostatic epithelium to hormonal influences, the peri-urethral (central) glands being more sensitive to androgen withdrawal than sub-capsular (peripheral) zones, as occurred in this study. Thus the prostatic changes seen are considered to reflect a pharmacological effect of the compound used rather than evidence of toxicity.

Mean absolute organ weights were adjusted to 100 g of the mean terminal body weights => mean relative organ weights.

Mean relative organ weights were compared to the corresponding control => % deviation.

**Table I**

| | TESTES | | | PROSTATE | | |
|---|---|---|---|---|---|---|
| Dose mg/kg/d | absolute | per 100g BW relative | diff. % vs. control | absolute | per 100g BW relative | diff. % vs. control |
| 0/veh | 24.006 | 0.1698 | (=100%) | 11.006 | 0.078 | (=100%) |
| 6 | 24.577 | 0.1803 | + 1% | 8.066 | 0.059 | - 24% |
| 20 | 23.432 | 0.1804 | + 6% | 6.413 | 0.049 | -37% |
| 60 | 24.753 | 0.1853 | + 9% | 4.429 | 0.033 | -58% |

Apart from the prostate changes no overt abnormalities in any other organ system was observed. Mild changes in the liver (hepatocyte hypertrophy with slightly increased organ weights) were considered to remain within the normal physiological adaptive range of dogs of this strain, with no signs of an overt systemic effect.

Preparation of 2-(3,5-Bis-trifluoromethyl-phenyl)-N-[6-(1,1-dioxo-1λ⁶-thiomorpholin-4-yl)-4-o-tolyl-pyridin-3-yl]-N-methyl-isobutyramide

### a) 4-(5-Nitro-pyridin-2-yl)-thiomorpholine

To a solution of 20 g (126 mmol) of 2-chloro-5-nitropyridine in 200 ml tetrahydrofuran were added dropwise 32.5 ml (315 mmol) thiomorpholine within 10 min. The reaction mixture was refluxed for additional 2 h. After cooling to room temperature, the solvent was removed *in vacuo* and the residue was re-dissolved in 200 ml ethyl acetate. The organic phase was washed with 200 ml 1N sodium bicarbonate solution, dried (magnesium sulfate) and evaporated to give 29.3 g (quantitative) of the title compound as a yellow solid.

MS m/e (%): 225 (M⁺, 78), 152 (100), 124 (62).

### b) 2,2-Dimethyl-N-(6-thiomorpholin-4-yl-pyridin-3-yl)-propionamide

To a suspension of 1.0 g (4.4 mmol) of 4-(5-nitro-2-pyridyl)-thiomorpholine in 8 ml ethanol and 2 ml water were added 1.5 g (27 mmol) of iron powder. A few drops of 3 N hydrochloric acid solution in diethyl ether were added and the reaction mixture was heated at 85 °C for 18 h. The suspension was filtered and the residue was washed 5 times with 10-ml portions of ethanol. The filtrate was evaporated *in vacuo* to give 870 mg of a purple oil.

This crude product was dissolved in 10 ml dichloromethane. Under stirring, 700 mg (6 mmol) of pivaloyl chloride and 860 mg (7 mmol) of N-ethyldiisopropylamine were added and the reaction mixture was stirred at room temperature overnight. Then, 30 ml water and 3 ml of 1 N hydrochloric acid solution were added to reach pH 1. The organic layer was separated and the aqueous layer was washed with 1 N hydrochloric acid solution, adjusted to pH 10 with sodium carbonate and extracted with dichloromethane. The organic layer was dried (sodium sulfate) and evaporated to give 630 mg (51 %) of the title compound as purple crystals.

MS m/e (%): 280 (M+H⁺, 100).

### c) N-(4-Iodo-6-thiomorpholin-4-yl-pyridin-3-yl)-2,2-dimethyl-propionamide

Under argon, a solution of 75 g (268 mmol) 2,2-dimethyl-N-(6-thiomorpholin-4-yl-pyridin-3-yl)-propionamide, 187 g (1.61 mol) N,N,N',N'-tetramethylethylenediamine and 85 g (604 mmol) 2,2,6,6,-tetramethylpiperidine in 750 ml tetrahydrofuran was cooled to -65 °C in a dry ice bath. Within 30 min, 805 ml (1.29 mol) ofa 1.6 N n-butyllithium solution in hexane were added dropwise. The reaction mixture was allowed to warm up to -15 °C and was stirred for 3 h at this temperature. After cooling again to -70°C, 354 g (1.40 mol) iodine (dissolved in 1000 ml tetrahydrofuran) were added dropwise during 2 h and stirring was continued for 1 h. The suspension was warmed to -60 °C and was poured into 1000 ml of 30 % sodium thiosulfate pentahydrate solution. Then, 750 ml *tert*-butyl methyl ether were added and the organic layer was separated. The aqueous layer was extracted three times with 750-ml portions of *tert*-butyl methyl ether and the combined organic layers were dried (sodium sulfate) and evaporated. Flash chromatography gave 68.9 g (63 %) of the title compound as light brown crystals.

MS m/e (%): 406 (M+H⁺, 100).

### d) 2,2-Dimethyl-N-(6-thiomorpholin-4-yl-4-o-tolyl-pyridin-3-yl)-propionamide

A mixture of 4.05 g (10.0 mmol) N-(4-iodo-6-thiomorpholin-4-yl-pyridin-3-yl)-2,2-dimethyl-propionamide, 54 ml toluene, 16 ml 2 N sodium carbonate solution, 347 mg (0.3 mmol) tetrakis(triphenylphosphine)palladium(0), 67 mg (0.3 mmol) palladium(II) acetate and 1.50 g (11.0 mmol) o-tolylboronic acid was heated under argon at 80 °C for 18 h. After cooling to room temperature, the aqueous phase was separated and washed twice with ethyl acetate. The combined organic layers were washed with 50 ml brine, dried (sodium sulfate) and evaporated. Purification by flash-chromatography gave 3.57 g (quantitative) of the title compound as a light brown solid.

MS m/e (%): 392 (M+Na⁺, 4), 370 (M+H⁺, 100).

### e) 6-Thiomorpholin-4-yl-4-o-tolyl-pyridin-3-ylamine

A suspension of 3.45 g (9.3 mmol) 2,2-dimethyl-N-(6-thiomorpholin-4-yl-4-o-tolyl-pyridin-3-yl)-propionamide in 95 ml 3 N hydrochloric acid solution was heated under argon at 110°C overnight. The reaction mixture was cooled to room temperature, washed with two 100-ml portions of diethyl ether and filtered over celite. The filtrate was diluted with 20 ml water and was adjusted to pH 11 by addition of 28 % sodium hydroxide solution under ice cooling. The product was extracted with three 100-ml portions of dichloromethane. The combined organic layers were washed with 50 ml brine, dried (sodium sulfate) and evaporated to give 2.53 g (95 %) of the title compound as a brown solid.

MS m/e (%): 286 (M+H⁺, 100).

### f) Methyl-(6-thiomorpholin-4-yl-4-o-tolyl-pyridin-3-yl)-amine

To a solution of 2.46 g (8.6 mmol) 6-thiomorpholin-4-yl-4-0-tolyl-pyridin-3-ylamine in 38 ml tetrahydrofuran were added 2.38 g (17 mmol) potassium carbonate (dissolved in 25 ml water) and 1.03 g (9.5 mmol) ethyl cloroformate. The reaction mixture was stirred for 1 h at room temperature and evaporated to remove tetrahydrofuran. The aqueous layer was extracted twice with 50-ml portions of dichloromethane and the organic layer was dried (sodium sulfate) and evaporated *in vacuo.* The residual oil was dissolved in 30 ml tetrahydrofuran and 7.4 ml (2.6 mmol) 3.5 M sodium bis(2-methoxyethoxy)aluminum hydride solution in toluene were added within 30 min. The reaction mixture was stirred at 50 °C overnight. After cooling to 0 °C, 7.5 ml 1 N sodium hydroxide solution were added dropwise. Tetrahydrofuran was removed *in vacuo* and 10 ml of water were added. The aqueous layer was extracted twice with 20-ml portions of dichloromethane and the combined organic layers were dried (sodium sulfate), evaporated and purified by flash chromatography to give 2.37 g (92 %) of the title compound as a yellow solid.

MS m/e (%): 300 (M+H⁺, 100).

### g) 2-(3,5-Bis-trifluoromethyl-phenyl)-N-methyl-N-(6-thiomorpholin-4-yl-4-o-tolyl-pyridin-3-yl)-isobutyramide

A solution of 2.32 g (7.7 mmol) methyl-(6-thiomorpholin-4-yl-4-o-tolyl-pyridin-3-yl)-amine and 1.50 g (11.6 mmol) N-ethyldiisopropylamine in 20 ml tetrahydrofuran was cooled in an ice bath and 2.72 g (8.5 mmol) 2-(3,5-bis-trifluoromethyl-phenyl)-2-methyl-propionyl chloride were added dropwise. The reaction mixture was stirred at room temperature overnight and evaporated *in vacuo.* The residue was suspended in 200 ml 1 N sodium carbonate solution and extracted three times with 200-ml portions of ethyl acetate. The combined organic layers were dried (sodium sulfate) and evaporated. The residue was crystallized from ethanol to give 3.60 g (80 %) of the title compound as white crystals.

MS m/e (%): 582 (M+H⁺, 100).

### h) 2-(3,5-Bis-trifluoromethyl-phenyl)-N-[6-(1,1-dioxo-1λ⁶-thiomorpholin-4-yl)-4-o-tolyl-pyridin-3-yl]-N-methyl-isobutyramide

To a solution of 1.00 g (1.72 mmol) 2-(3,5-bis-trifluoromethyl-phenyl)-N-methyl-N-(6-thiomorpholin-4-yl-4-o-tolyl-pyridin-3-yl)-isobutyramide in 10 ml methanol were added 1.59 g (2.58 mmol) OXONE®. After stirring for 2 days at room temperature, 5 ml 38 % sodium hydrogensulfite solution and 20 ml saturated sodium carbonate solution were added consecutively and methanol was removed *in vacuo.* The residue was diluted with 25 ml water and extracted with three 25-ml portions of dichloromethane. The combined organic layers were dried (sodium sulfate), purified by flash chromatography and crystallized from ethanol to give 980 mg (93 %) of the title compound as white crystals. M.p. 200-201°C.

MS m/e (%): 636 (M+Na⁺, 20), 614 (M+H⁺, 100).

### Preparation of 2-(3,5-Bis-trifluoromethyl-phenyl)-N-[6-(1,1-dioxo-1λ⁶-thiomorpholin-4-yl)-4-(4-fluoro-2-methyl-phenyl)-pyridin-3-yl] -N-methyl-isobutyramide

The title compound was obtained as white crystals in comparable yields according to the procedures described above for the preparation of 2-(3,5-bis-trifluoromethyl-phenyl)-N-[6-(1,1-dioxo-1λ⁶-thiomorpholin-4-yl)-4-o-tolyl-pyridin-3-yl]-N-methyl-isobutyramide using 4-fluoro-2-methyl-phenylboronic acid instead of o-tolylboronic acid in step d). M.p. 162.1-163.6°C.

## Claims

1. The use of a NK 1 receptor antagonist for the manufacture of a medicament for the treatment and/or prevention of benign prostatic hyperplasia wherein the NK-1 receptor antagonist is a compound of general formula (I) 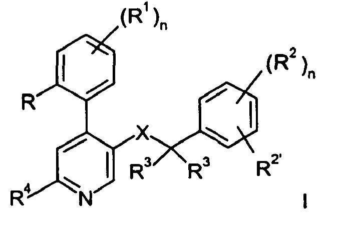 wherein
R is hydrogen, C₁₋₇-alkyl, C₁₋₇-alkoxy, halogen or trifluoromethyl;
R¹ is hydrogen or halogen; or
R and R¹ may be together -CH=CH-CH=CH-;
R² and R^{2'} are independently from each other hydrogen, halogen, trifluoromethyl, C₁₋₇-alkoxy or cyano; or
R² and R^{2'} may be together -CH=CH-CH=CH-, optionally substituted by one or two substituent selected from C₁₋₇-alkyl or C₁₋₇-alkoxy;
R³ is hydrogen, C₁₋₇-alkyl or form a cycloalkyl group;
R⁴ is hydrogen, -N(R⁵)₂, -N(R⁵)(CH₂)ₙOH, -N(R⁵)S(O)₂- C₁₋₇-alkyl, -N(R⁵)S(O)₂-phenyl, -N=CH-N(R⁵)₂, -N(R⁵)C(O)R⁵ or a cyclic tertiary amine of the group 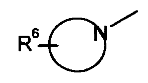 or the group 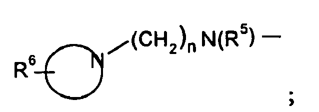
R⁵ is, independently from each other, hydrogen, C₃-C₆ cycloalkyl, benzyl or C₁₋₇-alkyl;
R⁶ is hydrogen, hydroxy, C₁₋₇-alkyl, -(CH₂)ₙCOO-C₁₋₇-alkyl, -N(R⁵)CO- C₁₋₇-alkyl, hydroxy-C₁₋₇-alkyl, cyano, -(CH₂)ₙO(CH₂)ₙOH, -CHO or a 5-or 6 membered heterocyclic group, optionally bonded via an alkylene group;
X is -C(O)N(R⁵)- or -N(R⁵)C(O)-, ;
n is 0,1,2,3or 4;
and the pharmaceutically acceptable acid addition salts thereof.

2. The use according to claim 1, wherein the NK-1 receptor antagonist is a compound of general formula (I), wherein X is -C(O)N(R⁵)- and R⁵ is methyl, ethyl or cyclopropyl.

3. The use according to claim 2, wherein the compound is selected from the group consisting of
N-(3,5-bis-trifluoromethyl-benzyl)-N-methyl-4-o-tolyl-nicotinamide,
N-(3,5-bis-trifluoromethyl-benzyl)-N-methyl-4-(2-chloro-phenyl)-nicotinamide,
N-(3,5-bis-trifluoromethyl-benzyl)-N-methyl-4-(2-trifluoromethyl-phenyl)-nicotinamide,
N-(3,5-bis-trifluoromethyl-benzyl)-N-methyl-4-(2-fluoro-phenyl)-nicotinamide,
N-(3,5-bis-trifluoromethyl-benzyl)-N-methyl-4-(2-methoxy-phenyl)-nicotinamide,
N-(3,5-bis-trilluoromethyl-benzyl)-N-methyl-4-phenyl-nicotinamide,
N-(3,5-bis-trifluoromethyl-benzyl)-N-ethyl-4-o-tolyl-nicotinamide,
N-(3,5-bis-trifluoromethyl-benzyl)-N-cyclopropyl-4-o-tolyl-nicotinamide,
N-[1-(3,5-bis-trifluoromethyl-phenyl)-ethyl]-N-methyl-4-o-tolyl-nicotinamide,
N-(3,5-di-fluorobenzyl)-N-methyl-4-o-tolyl-nicotinamide,
N-(3,5-di-chlorobenzyl)-N-methyl-4-o-tolyl-nicotinamide,
N-(3,5-bis-trifluoromethyl-benzyl)-N-methyl-6-(4-methyl-piperazin-1-yl)-4-o-tolyl-nicotinamide,
N-(3,5-bis-trifluoromethyl-benzyl)-N-methyl-6-(4-methyl-piperazin-1-yl)-4-naphthalen-1-yl-nicotinamide,
(4-{5-[(3,5-bis-trifluoromethyl-benzyl)-methyl-carbamoyl]-4-o-tolyl-pyridin-2-yl}-piperazin-1-yl)-acetic acid ethyl ester,
5'-[(3,5-bis-trifluoromethyl-benzyl)-methyl-carbamoyl]-4'-o-tolyl-3,4,5,6-tetrahydro-2H-[1,2']bipyridinyl-4-carboxylic acid ethyl ester,
N-(3,5-bis-trifluoromethyl-benzyl)-N-methyl-6-(4-propyl-piperazin-1-yl)-4-o-tolyl-nicotinamide,
(RS)-6-[3-(acetyl-methyl-amino)-pyrrolidin-1-yl]-N-(3,5-bis-trifluoromethylbenzyl)-N-methyl-4-o-tolyl-nicotinamide,
N-(3,5-bis-trifluoromethyl-benzyl)-N-methyl-6-[methyl-(2-morpholin-4-yl-ethyl)-amino] -4-o-tolyl-nicotinamide,
N-(3,5-bis-trifluoromethyl-benzyl)-N-methyl-6-morpholin-4-yl-4-o-tolyl-nicotinamide,
N-(3,5-bis-trifluoromethyl-benzyl)-N-methyl-6-thiomorpholin-4-yl-4-o-tolyl-nicotinamide,
N-(3,5-bis-trifluoromethyl-benzyl)-N-methyl-6-( 1-oxo-1λ⁴-thiomorpholin-4-yl)-4-o-tolyl-nicotinamide,
N-(3,5-bis-trifluoromethyl-benzyl)-6-(1,1-dioxo-1λ⁶-thiomorpholin-4-yl)-N-methyl-4-o-tolyl-nicotinamide,
N-(3,5-bis-trifluoromethyl-benzyl)-N-methyl-6-piperazin-1-yl-4-o-tolyl-nicotinamide,
N-(3,5-bis-trifluoromethyl-benzyl)-6-[4-(2-hydroxy-ethyl)-piperazin-1-yl]-N-methyl-4-o-tolyl-nicotinamide,
N-(3,5-bis-trifluoromethyl-benzyl)-6-(4-cyanomethyl-piperazin-1-yl)-N-methyl-4-o-tolyl-nicotinamide,
N-(3,5-bis-trifluoromethyl-benzyl)-6-{4-[2-(2-hydroxy-ethoxy)-ethyl] -piperazin-1-yl} -N-methyl-4-o-tolyl-nicotinamide,
N-(3,5-bis-trifluoromethyl-benzyl)-N-methyl-6-(4-[1,2,4]oxadiazol-3- yl-methyl-piperazin-1-yl)-4-o-tolyl-nicotinamide,
N-(3,5-bis-trifluoromethyl-benzyl)-N-methyl-6-[4-(5-oxo-4,5-dihydro-1H-[1,2,4] triazol-3-yl-methyl)-piperazin-1-yl]-4-o-tolyl-nicotinamide,
N-(3,5-bis-trifluoromethyl-benzyl)-6-(4-formyl-piperazin-1-yl)-N-methyl-4-o-tolyl-nicotinamide, and
N-methyl-N-(2-methyl-naphthalen-1-yl-methyl)-6-morpholin-4-yl-4-o-tolyl-nicotinamide;
or a pharmaceutically acceptable acid addition salt thereof.

4. The use according to claim 1, wherein the NK-1 receptor antagonist is a compound of general formula (I), wherein X is -N(R⁵)-C(O)- and R⁵ is hydrogen or methyl.

5. The use according to claim 4, wherein the compound is selected from the group consisting of
2-(3,5-bis-trifluoromethyl-phenyl)-N-methyl-N-[6-(4-methyl-piperazin-1-yl)-4-o-tolyl-pyridin-3-yl]-isobutyramide,
2-(3,5-bis-trifluoromethyl-phenyl)-N-[4-(2-chloro-phenyl)-6-(4-methyl-piperazin-1-yl)-pyridin-3-yl]-N-methyl-isobutyramide,
2-(3,5-bis-trifluoromethyl-phenyl)-N-[4-(4-fluoro-2-methyl-phenyl)-6-(4-methyl-piperazin-1-yl)-pyridin-3-yl]-N-methyl-isobutyramide,
2-(3,5-bis-trifluoromethyl-phenyl)-N-[4-(2-chloro-phenyl)-pyridin-3-yl]-N-methyl-isobutyramide,
2-(3,5-bis-trifluoromethyl-phenyl-N-methyl-N-(4-o-tolyl-pyridin-3-yl)-isobutyramide,
2-(3,5-bis-trifluoromethyl-phenyl)-N-(4-o-tolyl-pyridin-3-yl)-isobutyramide,
2-(3,5-bis-trifluoromethyl-phenyl)-N-methyl-N-(4-o-tolyl-pyridin-3-yl)-acetamide,
2-(3,5-bis-trifluoromethyl-phenyl)-N-methyl-N-(4-o-tolyl-pyridin-3-yl)-propionamide,
2-(3,5-bis-trifluoromethyl-phenyl)-N-methyl-N-(6-morpholin-4-yl-4-o-tolyl-pyridin-3-yl)-isobutyramide,
2-(3,5-bis-trifluoromethyl-phenyl)-N-[4-(2-chloro-phenyl)-6-morpholin-4-yl-pyridin-3-yl]-N-methyl-isobutyramide,
2-(3,5-bis-trifluoromethyl-phenyl)-N-methyl-N-[6-[methyl-(2-morpholin-4-yl-ethyl)-amino]-4-o-tolyl-pyridin-3-yl}-isobutyramide,
2-(3,5-bis-trifluoromethyl-phenyl)-N-methyl-N-[6-(4-pyrimidin-2-yl-piperazin-1-yl)-4-o-tolyl-pyridin-3-yl]-isobutyramide,
2-(3,5-bis-trifluoromethyl-phenyl)-N-(6-morpholin-4-yl-4-o-tolyl-pyridin-3-yl)-isobutyramide,
2-(3,5-bis-trifluoromethyl-phenyl)-N-[4-(2-chloro-phenyl)-6-dimethylamino-pyridin-3-yl] -isobutyramide,
2-(3,5-bis-trifluoromethyl-phenyl)-N-methyl-N-(6-piperazin-1-yl-4-o-tolyl-pyridin-3-yl)-isobutyramide,
2-(3,5-bis-trifluoromethyl-phenyl)-N-(4-hydroxy-4'-o-tolyl-3,4,5,6-tetrahydro-2H-[ 1,2']bipyridinyl-5'-yl)-N-methyl-isobutyramide,
2-(3,5-bis-trifluoromethyl-phenyl)-N-{6-[(2-hydroxy-ethyl)-methyl-amino]-4-o-tolyl-pyridin-3-yl}-N-methyl-isobutyramide,
(R)-2-(3,5-bis-trifluoromethyl-phenyl)-N-[6-(3-hydroxy-pyrrolidin-1-yl)-4-o-tolyl-pyridin-3-yl]-N-methyl-isobutyramide,
2-(3,5-bis-trifluoromethyl-phenyl)-N-methyl-N-(6-morpholin-4-yl-4-o-tolyl-pyridin-3-yl)-acetamide, and
[2-(3,5-bis-trifluoromethyl-phenyl)-2-methyl-propyl]-[4-(4-fluoro-2-methylphenyl)-6-(4-methyl-piperazin-1-yl)-pyridin-3-yl]-methylamine;
or a pharmaceutically acceptable acid addition salt thereof.

6. The use according to claim 1, wherein the NK-1 receptor antagonist is 2-(3,5-bis-trifluoromethyl-phenyl)-N-methyl-N-(6-morpholin-4-yl-4-o-tolyl-pyridin-3-yl)-isobutyramide or is a pharmaceutically acceptable acid addition salt thereof.

7. The use according to claim 1, wherein the NK-1 receptor antagonist is 2-(3,5-bis-trifluoromethyl-phenyl)-N-methyl-N-[6-(4-methyl-piperazin-1-yl)-4-o-tolyl-pyridin-3-yl]-isobutyramide, 2-(3,5-bis-trifluoromethyl-phenyl)-N-[6-(1,1-dioxo-1λ⁶-thiomorpholin-4-yl)-4-o-tolyl-pyridin-3-yl)-N-methyl-isobutyramide, or 2-(3,5-bis-trifluoromethyl-phenyl)-N-[6-(1,1-dioxo-1λ⁶-thiomorpholin-4-yl)-4-(4-fluoro-2-methyl-phenyl)-pyridin-3-yl]--N-methyl-isobutyramide or pharmaceutically acceptable acid addition salts thereof.

## Patentansprüche

1. Verwendung eines NK-1-Rezeptorantagonisten zur Herstellung eines Medikaments für die Behandlung und/oder Vorbeugung von benigner Prostatahyperplasie, worin der NK-1-Rezeptorantagonist eine Verbindung der allgemeinen Formel (I) ist 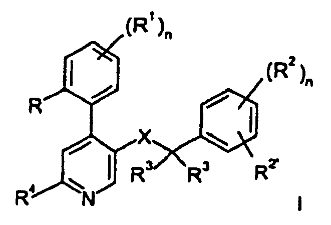 worin
R Wasserstoff, C₁₋₇-Alkyl, C₁₋₇-Alkoxy, Halogen oder Trifluormethyl ist;
R¹ Wasserstoff oder Halogen ist; oder
R und R¹ zusammen -CH=CH-CH=CH- sein können;
R² und R^{2'} unabhängig voneinander Wasserstoff, Halogen, Trifluormethyl, C₁₋₇-Alkoxy oder Cyano sind; oder
R² und R^{2'} zusammen -CH=CH-CH=CH- sein können, gegebenenfalls substituiert durch einen oder zwei Substituenten, ausgewählt aus C₁₋₇-Alkyl oder C₁₋₇-Alkoxy;
R³ Wasserstoff, C₁₋₇-Alkyl ist oder eine Cycloalkylgruppe bildet;
R⁴ Wasserstoff, -N(R⁵)₂, -N(R⁵)(CH₂)ₙOH, -N(R⁵)S(O)₂-C₁₋₇-Alkyl, -N(R⁵)S(O)₂-Phenyl, -N=CH-N(R⁵)₂, -N(R⁵)C(O)R⁵ oder ein cyclisches tertiäres Amin der Gruppe 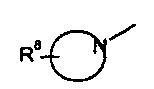 oder der Gruppe 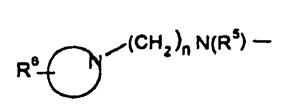 ist;
R⁵ unabhängig voneinander Wasserstoff, C₃-C₆-Cycloalkyl, Benzyl oder C₁₋₇-Alkyl ist;
R⁶ Wasserstoff, Hydroxy, C₁₋₇-Alkyl, -(CH₂)ₙCOO-C₁₋₇-Alkyl, -N(R⁵)CO-C₁₋₇-Alkyl, Hydroxy-C₁₋₇-alkyl, Cyano, -(CH₂)ₙO(CH₂)ₙOH, -CHO oder eine 5- oder 6-gliedrige heterocyclische Gruppe ist, die gegebenenfalls über eine Alkylengruppe gebunden ist;
X -C(O)N(R⁵)- oder -N(R⁵)C(O)- ist;
n 0, 1, 2, 3 oder 4 ist;
und der pharmazeutisch akzeptablen Säureadditionssalze davon.

2. Verwendung nach Anspruch 1, wobei der NK-1-Rezeptorantagonist eine Verbindung der allgemeinen Formel (I) ist, worin X -C(O)N(R⁵)- ist und R⁵ Methyl, Ethyl oder Cyclopropyl ist.

3. Verwendung nach Anspruch 2, wobei die Verbindung aus der Gruppe ausgewählt ist, bestehend aus
N-(3,5-Bis-trifluormethyl-benzyl)-N-methyl-4-o-tolyl-nicotinamid,
N-(3,5-Bis-trifluormethyl-benzyl)-N-methyl-4-(2-chlor-phenyl)-nicotinamid,
N-(3,5-Bis-trifluormethyl-benzyl)-N-methyl-4-(2-trifluormethyl-phenyl)-nicotinamid,
N-(3,5-Bis-trifluormethyl-benzyl)-N-methyl-4-(2-fluor-phenyl)-nicotinamid,
N-(3,5-Bis-trifluormethyl-benzyl)-N-methyl-4-(2-methoxy-phenyl)-nicotinamid,
N-(3,5-Bis-trifluormethyl-benzyl)-N-methyl-4-phenyl-nicotinamid,
N-(3,5-Bis-trifluormethyl-benzyl)-N-ethyl-4-o-tolyl-nicotinamid,
N-(3,5-Bis-trifluormethyl-benzyl)-N-cyclopropyl-4-o-tolyl-nicotinamid,
N-[1-(3,5-Bis-trifluormethyl-phenyl)-ethyl]-N-methyl-4-o-tolyl-nicotinamid,
N-(3,5-Di-fluorbenzyl)-N-methyl-4-o-tolyl-nicotinamid,
N-(3,5-Di-chlorbenzyl)-N-methyl-4-o-tolyl-nicotinamid,
N-(3,5-Bis-trifluormethyl-benzyl)-N-methyl-6-(4-methyl-piperazin-1-yl)-4-o-tolyl-nicotinamid,
N-(3,5-Bis-trifluormethyl-benzyl)-N-methyl-6-(4-methyl-piperazin-1-yl)-4-naphthalin-1-yl-nicotinamid,
(4- {5-[(3,5-Bis-trifluormethyl-benzyl)-methyl-carbamoyl]-4-o-tolyl-pyridin-2-yl) -piperazin-1-yl)-essigsäureester,
5'-[(3,5-Bis-trifluormethyl-benzyl)-methyl-carbamoyl]-4'-o-tolyl-3,4,5,6-tetrahydro-2H-[1,2']bipyridinyl-4-carbonsäureethylester,
N-(3,5-Bis-trifluormethyl-benzyl)-N-methyl-6-(4-propyl-piperazin-1-yl)-4-0-tolyl-nicotinamid,
(RS)-6-[3-(Acetyl-methyl-amino)-pyrrolidin-1-yl]-N-(3,5-bis-trifluormethyl-benzyl)-N-methyl-4-o-tolyl-nicotinamid,
N-(3,5-Bis-trifluormethyl-benzyl)-N-methyl-6-[methyl-(2-morpholin-4-yl-ethyl)-amino]-4-o-tolyl-nicotinamid,
N-(3,5-Bis-trifluormethyl-benzyl)-N-methyl-6-morpholin-4-yl-4-o-tolyl-nicotinamid,
N-(3,5-Bis-trifluormethyl-benzyl)-N-methyl-6-thiomorpholin-4-yl-4-o-tolyl-nicotinamid,
N-(3,5-Bis-trifluormethyl-benzyl)-N-methyl-6-( 1-oxo-1λ⁴-thiomorpholin-4-yl)-4-o-totyl-nicotinamid,
N-(3,5-Bis-trifluormethyl-benzyl)-6-(1,1-dioxo-1λ⁶-thiomorpholin-4-yl)-N-methyl-4-o-tolyl-nicotinamid,
N-(3,5-Bis-trifluormethyl-benzyl)-N-methyl-6-piperazin-1-yl-4-o-tolyl-nicotinamid,
N-(3,5-Bis-trifluormethyl-benzyl)-6-[4-(2-hydroxy-ethyl)-piperazin-1-yl]-N-methyl-4-o-tolyl-nicotinamid,
N-(3,5-Bis-trifluormethyl-benzyl)-6-(4-cyanomethyl-piperazin-1-yl)-N-methyl-4-o-tolyl-nicotinamid,
N-(3,5-Bis-trifluormethyl-benzyl)-6-{4-[2-(2-hydroxy-ethoxy)ethyl]-piperazin-1-yl}-N-methyl-4-o-tolyl-nicotinamid,
N-(3,5-Bis-trifluormethyl-benzyl)-N-methyl-6-(4-[1,2,4]oxadiazol-3-yl-methyl-piperazin-1-yl)-4-o-tolyl-nicotinamid,
N-(3,5-Bis-trifluormethyl-benzyl)-N-methyl-6-[4-(5-oxo-4,5-dihydro-1H-[1,2,4]triazol-3-yl-methyl)-piperazin-1-yl]-4-o-tolyl-nicotinamid,
N-(3,5-Bis-trifluormethyl-benzyl)-6-(4-formyl-piperazin-1-yl)-N-methyl-4-o-tolyl-nicotinamid und
N-Methyl-N-(2-methyl-naphthalin-1-yl-methyl)-6-morpholin-4-yl-4-o-tolyl-nicotinamid;
oder einem pharmazeutisch akzeptablen Säureadditionssalz davon.

4. Verwendung nach Anspruch 1, wobei der NK-1-Rezeptorantagonist eine Verbindung der allgemeinen Formel (I) ist, worin X -N(R⁵)-C(O)- ist und R⁵ Wasserstoff oder Methyl ist.

5. Verwendung nach Anspruch 4, wobei die Verbindung aus der Gruppe ausgewählt ist, bestehend aus
2-(3,5-Bis-trifluormethyl-phenyl)-N-methyl-N-[6-(4-methyl-piperazin-1-yl)-4-tolyl-pyridin-3-yl]-isobutyramid,
2-(3,5-Bis-trifluormethyl-phenyl)-N-[4-(2-chlor-phenyl)-6-(4-methyl-piperazin-1-yl)-pyridin-3-yl]-N-methyl-isobutyramid,
2-(3,5-Bis-trifluormethyl-phenyl)-N-[4-(4-fluor-2-methyl-phenyl)-6-(4-methyl-piperazin-1-yl)-pyridin-3-yl]-N-methyl-isobutyramid,
2-(3,5-Bis-trifluormethyl-phenyl)-N-[4-(2-chlor-phenyl)-pyridin-3-yl]-N-methyl-isobutyramid,
2-(3,5-Bis-trifluormethyl-phenyl)-N-methyl-N-(4-o-tolyl-pyridin-3-yl)-isobutyramid,
2-(3,5-Bis-trifluormethyl-phenyl)-N-(4-o-tolyl-pyridin-3-yl)-isobutyramid,
2-(3,S-Bis-trifluormethyl-phenyl)-N-methyl-N-(4-o-tolyl-pyridin-3-yl)-acetamid,
2-(3,S-Bis-trifluormethyl-phenyl)-N-methyl-N-(4-o-tolyl-pyridin-3-yl)-propionamid,
2-(3,5-Bis-trifluormethyl-phenyl)-N-methyl-N-(6-morpholin-4-yl-4-o-tolyl-pyridin-3-yl)-isobutyramid,
2-(3,5-Bis-trifluormethyl-phenyl)-N-[4-(2-chlor-phenyl)-6-morpholin-4-yl-pyridin-3-yl]-N-methyl-isobutyramid,
2-(3,5-Bis-trifluormethyl-phenyl)-N-methyl-N- {6-{methyl-(2-morpholin-4-yl-ethyl)-amino]-4-o-tolyl-pyridin-3-yl}-isobutyramid,
2-(3,5-Bis-trifluormethyl-phenyl)-N-methyl-N-[6-(4-pyrimidin-2-yl-piperazin-1-yl)-4-o-tolyl-pyridin-3-yl]-isobutyramid,
2-(3,5-Bis-trifluormethyl-phenyl)-N-(6-morpholin-4-yl-4-o-tolyl-pyridin-3-yl)isobutyramid,
2-(3,5-Bis-trifluormethyl-phenyl)-N-[4-(2-chlor-phenyl)-6-dimethylamino-pyridin-3-yl]-isobutyramid,
2-(3,5-Bis-trifluormethyl-phenyl)-N-methyl-N-(6-piperazin-1-yl-4-o-tolyl-pyridin-3-yl)-isobutyramid,
2-(3,5-Bis-trifluormethyl-phenyl)-N-(4-hydroxy-4'-o-tolyl-3,4,5,6-tetrahydro-2H-[1,2']bipyridinyl-5'-yl)-N-methyl-isobutyramid,
2-(3,5-Bis-trifluormethyl-phenyl)-N-{6-[(2-hydroxy-ethyl)-methyl-amino]-4-o-tolyl-pyridin-3-yl}-N-methyl-isobutyramid,
(R)-2-(3,S-Bis-trifluormethyl-phenyl)-N-[6-(3-hydroxy-pyrrolidin-1-yl)-4-o-tolyl-pyridin-3-yl]-N-methyl-isobutyramid,
2-(3,5-Bis-trifluormethyl-phenyl)-N-methyl-N-(6-morpholin-4-yl-4-o-tolyl-pyridin-3-yl)-acetamid und
[2-(3,5-Bis-trifluormethyl-phenyl)-2-methyl-propyl]-[4-(4-fluor-2-methyl-phenyl)-6-(4-methyl-piperazin-1-yl)-pyridin-3-yl]-methylamin;
oder einem pharmazeutisch akzeptablen Säureadditionssalz davon.

6. Verwendung nach Anspruch 1, wobei der NK-1-Rezeptorantagonist 2-(3,5-Bis-trifluormethyl-phenyl)-N-methyl-N-(6-morpholin-4-yl-4-o-tolyl-pyridin-3-yl)-isobutyramid oder ein pharmazeutisch akzeptables Säureadditionssalz davon ist.

7. Verwendung nach Anspruch 1, wobei der NK-1-Rezeptorantagonist 2-(3,5-Bis-trifluormethyl-phenyl)-N-methyl-N-[6-(4-methyl-piperazin-1-yl)-4-o-tolyl-pyridin-3-yl]-isobutyramid, 2-(3,5-Bis-trifluormethyl-phenyl)-N-[6-(1,1-dioxo-1λ⁶-thiomorpholin-4-yl)-4-o-tolylpyridin-3-yl]-N-methyl-isobutyramid oder 2-(3,5-Bis-trifluormethyl-phenyl)-N-[6-(1,1-dioxo-1 λ⁶-thiomorpholin-4-yl)-4-(4-fluor-2-methyl-phenyl)-pyridin-3-yl]-N-methyl-iso-butyramid oder pharmazeutisch akzeptable Säureadditionssalze davon ist.

## Revendications

1. Utilisation d'un antagoniste de récepteur de NK-1 pour la fabrication d'un médicament pour le traitement et/ou la prévention de l'hyperplasie bénigne de la prostate dans laquelle l'antagoniste de récepteur de NK 1 est un com-posé de formule générale (I) 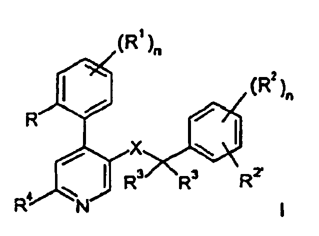 dans laquelle
R est un hydrogène, un alkyle en C₁ à C₇, alcoxy en C₁ à C₇, halogène ou trifluorométhyle ;
R¹ est un hydrogène ou un halogène ; ou
R et R¹ peuvent être ensemble -CH=CH-CH=CH- ;
R² et R^{2'} sont indépendamment l'un de l'autre un hydrogène, halogène, trifluorométhyle, alcoxy en C₁ à C₇ ou cyano; ou
R² et R^{2'} peuvent être ensemble -CH=CH-CH=CH-, éventuellement substitué par un ou deux substituants choisis parmi un alkyle en C₁ à C₇ ou alcoxy en C₁ à C₇ ;
R³ est un hydrogène, un alkyle en C₁ à C₇ ou forme un groupe cycloalkyle ;
R⁴ est un hydrogène, -N(R⁵)₂, -N(R⁵) (CH₂)ₙ₋OH, -N(R⁵)S(O)₂-(alkyle en C₁ à C₇), -N-(R⁵)S(O)₂-phényle, -N=CH-N (R⁵)₂, -N(R⁵)C-(O)R⁵ ou une amine tertiaire du groupe 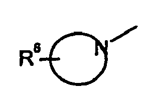
ou le groupe 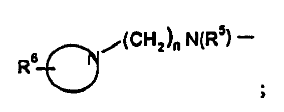
R⁵ est, indépendamment des autres, un hydrogène, un cycloalkyle en C₃ à C₆, benzyle ou alkyle en C₁ à C₇ ;
R⁶ est un hydrogène, hydroxy, alkyle en C₁ à C₇, -(CH₂)ₙCOO-(alkyle en C₁ à C₇), -N-(R⁵) CO- (alkyle en C₁ à C₇), hydroxy-(alkyle en C₁ à C₇), cyano, -(CH₂)ₙO-(CH₂)ₙOH, -CHO ou un groupe hétérocyclique à 5 ou 6 chaînons, éventuellement liés via un groupe alkylène ;
X est -C(O)N(R⁵)- ou -N(R⁵)C(O)-
n est 0, 1, 2, 3 ou 4 ;
et leurs sels d'addition avec un acide pharmaceutiquement acceptables.

2. Utilisation selon la revendication 1, dans laquelle l'antagoniste de récepteur de NK-1 est un composé de formule générale (I) dans laquelle X est -C(O)N (R⁵) - et R⁵ est un méthyle, éthyle ou cyclopropyle.

3. Utilisation selon la revendication 2, dans laquelle on choisit le composé parmi le groupe constitué de
N-(3,5-bis-trifluorométhyl-benzyl)-N-méthyl-4-o-tolyl-nicotinamide,
N-(3,5-bis-trifluorométhyl-benzyl)-N-méthyl-4-(2-chloro-phényl)-nicotinamide,
N-(3,5-bis-trifluorométhyl-benzyl)-N-méthyl-4-(2-trifluorométhyl-phényl)-nicotinamide,
N-(3,5-bis-trifluorométhyl-benzyl)-N-méthyl-4-(2-fluoro-phényl)-nicotinamide,
N-(3,5-bis-trifluorométhyl-benzyl)-N-méthyl-4-(2-méthoxy-phényl)-nicotinamide,
N-(3,5-bis-trifluorométhyl-benzyl)-N-méthyl-4-phényl-nicotinamide,
N-(3,5-bis-trifluorométhyl-benzyl)-N-éthyl-4-o-tolyl-nicotinamide,
N-(3,5-bis-trifluorométhyl-benzyl)-N-cyclopropyl-4-o-tolyl-nicotinamide,
N-[1-(3,5-bis-trifluorométhyl-phényl)éthyl]-N-méthyl-4-o-tolyl-nicotinamide,
N-(3,5-di-fluorobenzyl)-N-méthyl-4-o-tolyl-nicotinamide,
N-(3,5-di-chlorobenzyl)-N-méthyl-4-o-tolyl-nicotinamide,
N-(3,5-bis-trifluorométhyl-benzyl)-N-méthyl-6-(4-méthyl-pipérazin-1-yl)-4-o-tolyl-nicotinamide,
N-(3,5-bis-trifluorométhyl-benzyl)-N-méthyl-6-(4-méthyl-pipérazin-1-yl)-4-naphtalén-1-yl-nicotinamide,
ester éthylique d'acide (4-[5-[(3,5-bis-trifluorométhyl-benzyl)-méthyl-carbamoyl]-4-o-tolyl-pyridin-2-yl]-pipérazin-1-yl)-acétique,
ester éthylique d'acide 5'-[(3,5-bis-trifluorométhyl-benzyl)-méthyl-carbamoyl]-4'-o-tolyl-3,4,
5,6-tétrahydro-2H-[1,2']bipyridinyl-4-carboxylique,
N-(3,5-bis-trifluorométhyl-benzyl)-N-méthyl-6-(4-propyl-pipérazin-1-yl)-4-o-tolyl-nicotinamide.
(RS)-6-[3-acétyl-méthyl-amino)-pyrrolidin-1-yl]-N-(3,5-bis-trifluorométhyl-benzyl)-N-méthyl-4-o-tolyl-nicotinamide,
N-(3,5-bis-trifluorométhyl-benzyl)-N-méthyl-6-[méthyl-(2-morpholin-4-yl-éthyl)-amino]-4-o-tolyl-nicotinamide,
N-(3,5-bis-trifluorométhyl-benzyl)-N-méthyl-6-morpholin-4-yl-4-o-tolyl-nicotinamide,
N-(3,5-bis-trifluorométhyl-benzyl)-N-méthyl-6-thiomorpholin-4-yl-4-o-tolyl-nicotinamide,
N-(3,5-bis-trifluorométhyl-benzyl)-N-méthyl-6-(1-oxo-1λ⁴-thiomorpholin-4-yl)-4-o-tolyl-nicotinamide,
N-(3,5-bis-trifluorométhyl-benzyl)-6-(1,1-dioxo-1λ⁶-thiomorpholin-4-yl)-N-méthyl-4-o-tolyl-nicotinamide,
N-(3,5-bis-trifluorométhyl-benzyl)-N-méthyl-6-pipérazin-1-yl-4-o-tolyl-nicotinamide,
N-(3,5-bis-trifluorométhyl-benzyl)-6-[4-(2-hydroxy-éthyl)-pipérazin-1-yl]-N-méthyl-4-o-tolyl-nicotinamide,
N-(3,5-bis-trifluorométhyl-benzyl)-6-(4-cyanométhyl-pipérazin-1-yl)-N-méthyl-4-o-tolyl-nicotinamide,
N-(3,5-bis-trifluorométhyl-benzyl)-6-{4-[2-(2-hydroxy-éthoxy)-éthyl]-pipérazin-1-yl}-N-méthyl-4-o-tolyl-nicotinamide,
N-(3,5-bis-trifluorométhyl-benzyl)-N-méthyl-6-(4-[1,2,4]oxadiazol-3-yl-méthyl-pipérazin-1-yl)-4-o-tolyl-nicotinamide,
N-(3,5-bis-trifluorométhyl-benzyl)-N-méthyl-6-[4-(5-oxo-4,5-dihydro-1H-[1,2,4]triazol-3-yl-méthyl)-pipérazin-1-yl]-4-o-tolyl-nicotinamide,
N-(3,5-bis-trifluorométhyl-benzyl)-6-(4-formyl-pipérazin-1-yl)-N-méthyl-4-o-tolyl-nicotinamide,
et
N-méthyl-N-(2-méthyl-naphtalén-1-yl-méthyl)-6-morpholin-4-yl-4-o-tolyl-nicotinamide ;
ou leurs sels d'addition avec un acide pharmaceutiquement acceptables.

4. Utilisation selon la revendication 1, dans laquelle l'antagoniste de récepteur de NK-1 est un composé de formule générale (I), dans laquelle X est -N(R⁵)-C(O)- et R⁵ est un hydrogène ou un méthyle.

5. Utilisation selon la revendication 4, dans laquelle le composé est choisi dans le groupe constitué de
2-(3,5-bis-trifluorométhyl-phényl)-N-méthyl-N-[6-(4-méthyl-pipérazin-1-yl)-4-o-tolyl-pyridin-3-yl]-isobutyramide,
2-(3,5-bis-trifluorométhyl-phényl)-N-[4-(2-chloro-phényl)-6-(4-méthyl-pipérazin-1-yl)-pyridin-3-yl]-N-méthyl-isobutyramide,
2- (3, 5-bis-trifluorométhyl-phényl) -N- [4- (4-fluoro-2-méthyl-phényl)-6-(4-méthylpipérazin-1-yl)-pyridin-3-yl]-N-méthyl-isobutyramide,
2-(3,5-bis-trifluorométhyl-phényl)-N-[4-(2-chloro-phényl)-pyridin-3-yl]-N-méthyl-isobutyramide,
2-(3,5-bis-trifluorométhyl-phényl-N-méthyl-N-(4-o-tolyl-pyridin-3-yl)-isobutyramide,
2-(3,5-bis-trifluorométhyl-phényl)-N-(4-o-tolyl-pyridin-3-yl)-isobutyramide,
2-(3,5-bis-trifluorométhyl-phényl)-N-méthyl-N-(4-o-tolyl-pyridin-3-yl)-acétamide,
2-(3,5-bis-trifluorométhyl-phényl)-N-méthyl-N-(4-o-tolyl-pyridin-3-yl)-propionamide,
2-(3,5-bis-trifluorométhyl-phényl)-N-méthyl-N-(6-morpholin-4-yl-4-o-tolyl-pyridin-3-yl)-isobutyramide,
2- (3, 5-bis-trifluorométhyl-phenyl) -N [-4- (2-chloro-phényl)-6-morpholin-4-yl-pyridin-3-yl]-N-méthyl-isobutyramide,
2-(3,5-bis-trifluorométhyl-phényl)-N-méthyl-N-{6-[méthyl-(2-morpholin-4-yl-éthyl)-amino]-4-0-tolyl-pyridin-3-yl}-isobutyramide,
2-(3,5-bis-trifluorométhyl-phényl)-N-méthyl-N-[6-(4-pyrimidin-2-yl-pipérazin-1-yl)-4-o-tolyl-pyridin-3-yl]-isobutyramide,
2-(3,5-bis-trifluorométhyl-phényl)-N-(6-morpholin-4-yl-4-o-tolyl-pyridin-3-yl)-isobutyramide,
2-(3,5-bis-trifluorométhyl-phényl)-N-[4-(2-chloro-phényl)-6-diméthylaminopyridin-3-yl]-isobutyramide,
2-(3,5-bis-trifluorométhyl-phényl)-N-méthyl-N-(6-pipérazin-1-yl-4-o-tolylpyridin-3-yl)-isobutyramide,
2-(3,5-bis-trifluorométhyl-phényl)-N-(4-hydroxy-4'-o-tolyl-3,4,5,6-tétrahydro-2H-[1,2']bipyridinyl-5'-yl)-N-méthyl-isobutyramide,
2-(3,5-bis-trifluorométhyl-phényl)-N-{6-[(2-hydroxy-ethyl)-méthyl-amino]-4-o-tolyl-pyridin-3-yl}-N-méthyl-isobutyramide,
(R)-2-(3,5-bis-trifluorométhyl-phényl)-N-[6-(3-hydroxy-pyrrolidin-1-yl)-4-o-tolylpyridin-3-yl]-N-méthyl-isobutyramide,
2-(3,5-bis-trifluorométhyl-phényl)-N-méthyl-N-(6-morpholin-4-yl-4-o-tolyl-pyridin-3-yl)-acétamide, et
[2-(3,5-bis-trifluorométhyl-phényl)-2-méthyl-propyl] - [4- (4-fluoro-2-méthylphenyl) -6- (4-méthyl-pipérazin-1-yl)-pyridin-3-yl]-méthylamine;
ou un sel d'addition avec un acide pharmaceutiquement acceptable de ceux-ci.

6. Utilisation selon la revendication 1, dans laquelle l'antagoniste de récepteur de NK-1 est le 2-(3,5-bis-trifluorométhyl-phényl)-N-méthyl-N-(6-morpholin-4-yl-4-o-tolyl-pyridin-3-yl)-isobutyramide ou est un sel d'addition avec un acide pharmaceutiquement acceptable de celui-ci.

7. Utilisation selon la revendication 1, dans laquelle l'antagoniste de récepteur de NK-1 est le 2-(3,5-bis-trifluorométhyl-phényl)-N-méthyl-N- [6- (4-méthyl-pipérazin-1-yl) -4-o-tolyl-pyridin-3-yl]-isobutyramide, le 2-(3,5-bis-trifluorométhyl-phényl) -N-[6-(1,1-dioxo-1λ⁶⁻thiomorpholin-4-yl)-4-o-tolyl-pyridin-3-yl]-N-méthyl-isobutyramide, ou le 2-(3,5-bis-trifluorométhyl-phényl)-N-[6-(1,1-dioxo-1λ⁶⁻thiomorpholin-4-yl)-4-(4-fluoro-2-méthyl-phényl)-pyridin-3-yl]-N-méthyl-isobutyramide ou des sels d'addition avec un acide pharmaceutiquement acceptables de ceux-ci.
